Europäisches Patentamt

European Patent Office

Office européen des brevets

(11) Veröffentlichungsnummer: **0 440 100 B1**

## (12) EUROPÄISCHE PATENTSCHRIFT

(45) Veröffentlichungstag der Patentschrift: **19.10.94**

(21) Anmeldenummer: **91100950.4**

(22) Anmeldetag: **25.01.91**

(51) Int. Cl.5: **A61K 47/44**, A61K 9/08,
A61K 9/127, A61K 37/02,
A61K 37/66, A61K 45/05

(54) **Verwendung eines Gallensäuresalzes und eines Lipids.**

(30) Priorität: **25.01.90 CH 234/90**

(43) Veröffentlichungstag der Anmeldung:
**07.08.91 Patentblatt 91/32**

(45) Bekanntmachung des Hinweises auf die
Patenterteilung:
**19.10.94 Patentblatt 94/42**

(84) Benannte Vertragsstaaten:
**AT BE CH DE DK FR GB IT LI LU NL SE**

(56) Entgegenhaltungen:
EP-A- 439 042          EP-A- 0 056 781
EP-A- 0 263 493       EP-A- 0 280 492
EP-A- 0 280 503       WO-A-83/00294
DE-A- 2 730 570

JOURNAL OF CELLULAR BIO- CHEMISTRY,
Supplement 12A, January 17-30, 1988, NewY-
ork P. M. ANDERSON et al. "Lipo- somal
Interleukin 2 Synthesis and BiologicalActivity" Seite 255

(73) Patentinhaber: **F. HOFFMANN-LA ROCHE AG**
**Postfach 3255**
**CH-4002 Basel (CH)**

(72) Erfinder: **Ferro, Alberto, Dr.**
**Gstaltenrainweg 67**
**CH-4125 Riehen (CH)**
Erfinder: **Steffen, Hans, Dr.**
**Seestrasse 16**
**CH-4410 Liestal (CH)**
Erfinder: **Supersaxo, Andreas, Dr.**
**21230 Homestead Road 104**
**Cupertino, CA 95014 (US)**

(74) Vertreter: **Lederer, Franz, Dr. et al**
**Lederer, Keller & Riederer**
**Patentanwälte**
**Prinzregentenstrasse 16**
**D-80538 München (DE)**

JOURNAL OF CELLULAR BIO- CHEMISTRY, Supplement 12A, January 17-30, 1988, NewYork B BARNA et al. "Monocyte Tumoricidal Activity in Brain Tumors: In vitroActivation by Liposomal C-Reactive Protein (CRP)" Seite 255

JOURNAL OF CELLULAR BIO- CHEMISTRY, Supplement 12A, January 17-30, 1988, NewYork J. D. GANGEMI et al. "Anti- viral Properties of free and Liposome-Encapsulated Muramyl Tripeptide" Seite 255

PHARMAZIE, Band 44, 1989, Berlin J. FICHTNER, D. ARNDT "Stand und Perspektivender Lipo- somenforschung" Seiten 752-757

PATENT ABSTRACTS OF JAPAN, unexamined applications, C section, vol. 2, no. 89,July 21, 1978 THE PATENT OFFICE JAPANESE GOVERNMENT Seite 1430 C 78

**Beschreibung**

Die Erfindung betrifft die Verwendung eines Gallensäuresalzes und eines Lipids zur Adsorptionsverhinderung, Desorption, Agglomerationsverhinderung und Desagglomerierung immunmodulatorisch wirksamer Proteine in wässrigen Lösungen.

Der hier verwendete Ausdruck "immunmodulatorisch wirksames Protein" bezeichnet Proteine, die die Reifung, Aktivierung und Suppression der verschiedenen Zellen des menschlichen und tierischen Immunsystems regulieren. Die zum Einsatz kommenden Proteine können entweder natürlichen Ursprungs oder auf rekombinantem Weg hergestellt sein.

Beispiele solcher Proteine sind Interferone (IFN), wie IFN-$\alpha$, IFN-$\beta$, IFN-$\gamma$ Hybridinterferone; Interleukine (IL), wie IL-1 (ETAF, LAF), IL-2 (TCGF), IL-3 (Multi-CSF, MCGF), IL-4 (BSF-1, BCGF-1), IL-5 (TRF, BCGF-II), IL-7 (Lymphopoietin 1); Lymphotoxin (TNF-$\beta$); Macrophage Inhibitory Factor (MIF); Thymopoietin (TPO); Transforming Growth Factor-$\alpha$ (TGF-$\alpha$); Transforming Growth Factor-$\beta$ (TGF-$\beta$); Tumor Nekrose Faktor (TNF-$\alpha$, Cachectin, DIF); Uromodulin (Tamm-Horsfall-Protein); Neuroleukin; CD4.

Weitere Beispiele von Proteinen mit Wirkung auf Immunfunktionen sind Wachstums- und Differenzierungsfaktoren, wie Granulocyte Colony Stimulating Factor (G-CSF), Granulocyte-Macrophage Colony Stimulating Factor (GM-CSF, CSF-2), Macrophage Colony Stimulating Factor (CSF-1, M-CSF); Antikörper oder Antikörper-Arzneistoff-Konjugate, Hybridproteine wie IL-2-Diphtherietoxin und Proteine zur Herstellung von Vakzinen gegen AIDS, Malaria, Hepatitis, Herpes, Influenza, Poliomyelitis und andere Infektionskrankheiten.

Es wurde gefunden, dass sich solche Proteine mit wassrigen Lösungen von Gällensäurederivaten und Lipiden stabilisieren lassen und dass die so erhaltenen Mischmicell-Lösungen gegenüber konventionellen wässrigen Lösungen anwendungstechnische Vorteile mit sich bringen, z.B. grösseres Solubilisierungsvermögen, geringere Adsorption der Proteine an Oberflächen, geringere Aggregationsneigung und damit Herstellbarkeit konzentrierter Präparate, genauere Dosierbarkeit und bessere Lagerstabilität.

Aus dem Dokument J.Cell.Biochem.Suppl. 12A, UCLA Symposia 17-30 Januar 1988, Seite 255, Abstrakt No. W 100 sind liposomale Lösungen bekannt, die Interleukin 2, Natriumcholat, Dimyristoylphosphorylchol in und Dimyristoylphosphorylglycin enthalten. Das Dokument EP-A-56781 beschreibt liposomale Arzneimittel, die aus Phospholipiden, Cholsäurederivaten und Antigenen Zusammengesetzt sein können.

Die erfindungsgemässe Verwendung kann in an sich bekannter Weise, z.B. nach den in der DE-OS 2 730 570 angegebenen Methoden erfolgen.

Als Gallensäurederivate kommen in den erfindungsgemässen Präparaten die in der DE-OS 2 730 570 erwähnten Salze von Gallensäuren bzw. Gallensäurederivaten in Betracht, wie Cholate, Glycocholate und Taurocholate, insbesondere die Alkalisalze, wie die Natriumsalze. Besonders bevorzugt ist Na-Glycocholat.

Beispiele von Lipiden sind natürliche, semisynthetische oder vollsynthetische Phosphatidylcholine, Phosphatidyläthanolamin, Phosphatidyläthanolamin, Phosphatidylinositol, Phosphatidylserine, Sphingomyelin, Plasmalogene, Cardiolipin, Sulfatide, synthetische Lecithine mit abgewandelten Seitenketten z.B. solche, die in der europäischen Patentanmeldung EP-A-0154977 beschrieben sind. Die Mischmizellen können als zusätzliche Komponenten Cholesterin (bis etwa 30 Mol-% bezogen auf den Lipidanteil) und Lipide mit negativ oder positiv geladenen Gruppen enthalten, wie Phosphatidsäure oder Stearylamin (bis etwa 10 Mol-% bezogen auf den Lipidanteil).

Das Molverhältnis zwischen Lipid und Gallensäuresalz liegt zweckmässig in der Grössenordnung von 0,1:1 bis 2:1. Bevorzugt sind Mischungsverhältnisse von 0,8:1 bis 1,5:1.

Die Gesamtmenge von Lipid und Gallensäuresalz beträgt vorzugsweise etwa 50-300 mg/ml. Die Menge des Proteins pro Volumeneinheit in den Präparaten kann in weiten Bereichen variieren und hängt von der biologischen Aktivität des betreffenden Proteins ab. Im allgemeinen wird die Konzentration des Proteins etwa 0,001 mg/ml bis 10 mg/ml betragen, vorzugsweise etwa 0,01 bis 1 mg/ml.

Zur Verhinderung von Oxidationsreaktionen des Wirkstoffs und der Trägermaterialien können Antioxidantien, wie Tocopherole, Ascorbylpalmitat, Natriumascorbat, Natriumhydrogensulfit, Natriumpyrosulfit oder Natriumsulfit zugesetzt werden.

Weitere Hilfsstoffe zur pH-Einstellung, z.B. Phosphat-, Citrat- oder Tris-Puffer; zur Isotonisierung, z.B. Natriumchlorid, Mannitol, Sorbitol oder Glucose und zur Konservierung, z.B. Methyl- und Propyl-p-Hydroxybenzoesäure, Benzylalkohol oder Phenol können ebenfalls zugegeben werden.

Falls erwünscht, lassen sich die Mischmicell-Protein-Lösungen mit Hilfe konventioneller Trocknungsverfahren, wie z.B. Lyophilisation, in Trockenpräparate überführen.

Die erfindungsgemäss erhältlichen Präparate können parenteral, z.B. intravenös oder interstitiell, oder enteral, z.B. oral, nasal, buccal, rektal oder vaginal, oder topisch angewandt werden.

Die nachstehenden Beispiele erläutern die erfindungsgemässe Verwendung:

Beispiel 1

Lecithin (PC) und Na-Glycocholat (NaGC) werden im Molverhältnis 1:1 in Chloroform/Methanol (1:1 Vol.-Teile) gelöst. Die Lösungsmittel werden in einem rotierenden Rundkolben unter vermindertem Druck bei 40°C abgedampft. Der als Rückstand auf der Kolbenwandung verbleibende Film wird in Wasser dispergiert und unter Rühren mit dem Protein versetzt. Rekombinantes Interleukin 2 (rIL-2) und rekombinantes β-Interferon (rIFN-β) wurden als wässrige Pufferlösungen zugesetzt (die Zusammensetzung ist in untenstehender Tabelle 1 angegeben). His6-p190(I-2) (Plasmodium falciparum Oberflächenprotein) und HIV 22 (HIV 1 Fusionsprotein) wurden in lyophilisierter Form hinzugefügt. Die so erhaltenen Lösungen (PC- und NaGC-Konzentration je 100 mM) wurden durch Zusatz von 0,1 N NaOH auf pH 7,1 ± 0,1 eingestellt, mit $N_2$ inertbegast, sterilfiltriert (0,22 μm Millipore-Filter), in Ampullen abgefüllt und bei Raumtemperatur aufbewahrt.

Tabelle I

| Zusammensetzung der zur Herstellung der Micell-Lösung verwendeten Protein-Lösungen | | | |
|---|---|---|---|
| **Protein** | **Protein-Konzentration [mg/ml]** | **Puffer** | **pH** |
| rIL-2 | 3,4 | 50 mM Acetat/Puffer, 50 mg/ml Mannit | 3,5 |
| rIFN-β | 1,19 | 1 mM Citronensäure | |

Die so hergestellten Micell-Lösungen wurden auf ihr Solubilisierungsverhalten im Vergleich zu rein wässrigen (nicht-micellaren) Lösungen geprüft. Dazu wurden diese Lösungen 24 Stunden nach der Herstellung 1 Stunde bei 15°C ultrazentrifugiert (35000 U/Min.). Danach wurde der Proteingehalt nach Markwell (Analytical Biochemistry 87: 206-210 (1978) im Ueberstand gemessen. Die erhaltenen Messwerte sind in Tabelle II wiedergegeben:

Tabelle II

| Protein | Initial-Konzentration [mg/ml] | Proteingehalt nach Micell-Lösung [%] | Ultrazentrifugation wässrige Lösung [%][1] |
|---|---|---|---|
| rIL-2 | 1,00 | 91,0 ± 3,6 (n = 3) | 78,5 ± 11,5 (n = 3) |
| rIFN-β | 0,20 | 86,5 ± 2,25 (n = 3) | 27,3 ± 2,9 (n = 2) |
| His6-p190(I-2) | 0,20 | 75,9 ± 4,24 (n = 2) | 0,0 (n = 1) |
| HIV 22 | 1,00 | 97,0 ± 3,32 (n = 4) | 55,7 ± 6,3 (n = 4) |

1) % der Initial-Konzentration

Die Messwerte zeigen, dass rIL-2, rIFN-β, His6-p190(1-2) und HIV 22 in Mischmicell-Lösungen in deutlich höherer Konzentration solubilisiert in Lösung bleiben als in nicht micellaren, konventionellen wässrigen Lösungen. Dieser Effekt, der durch geringere Aggregations- und Adsorptionsneigung der Proteine in micellaren Lösungen erklärt werden kann, ist von erheblicher praktischer Bedeutung. Immunmodulatorisch wirksame Proteine sind extrem hochaktive Wirkstoffe. Die therapeutische Verwendung solcher Wirkstoffe setzt eine zuverlassige Dosierung voraus. Wie die Daten der Tabelle II zeigen, kann eine genauere Dosierung mit der erfindungsgemässen Lösung eindeutig zuverlässiger sichergestellt werden, als mit konventionellen Lösungen. Des weiteren kann eine durch Proteinaggregate hervorgerufene Immunantwort (Antikörperbildung) vermindert oder verhindert werden.

Beispiel 2

rIFN-α-Lösungen, welche aus Humanserumalbuminhaltigen resp. -freien Lyophilisaten mit 18 Mio. I.E. rIFN-α und 3 ml Wasser zu Injektionszwecken resp. 0,9 % Benzyl Alkohol rekonstituiert werden, zeigen innerhalb weniger Tage, in der Regel innerhalb 1-2 Tagen bei Raumtemperatur und innerhalb 2-5 Tagen bei 5°C, deutliche Agglomerate.

rIFN-α-Lösungen, welche aus denselben Lyophilisaten und einem Solvens hergestellt werden, das Natriumglycocholat und Lezithin enthält, bleiben dagegen bis mindestens 1 Monat nach Herstellung bei Raumtemperatur und bei 5°C physikalisch stabil und zeigen keine Verluste an antiviraler Aktivität (siehe Tabelle III).

Zur Bestimmung der antiviralen Aktivität des r-α-IFN wurde ein cytopathologischer Test mit MDBK (Madison Darby Bovine Kidney)-Zellen und VSV (vesicular stomatitis virus)-Viren eingesetzt, der von Rubinstein et al. [J.Virol. 37, 755-758(1981)] beschrieben worden ist.

## Tabelle III

Stabilität von α-Interferon-Lösungen aus Lyophilisaten mit 18 Mio I.E. rIFN-α und 3 ml Solvens

| Solvens | Physikalische Stabilität | Antivirale Aktivität (% des Anfangswertes) |
|---|---|---|
| Wasser zu Injektions zwecken | deutliche Partikelbildung (Wirbel aus dem Boden) nach 1-2 Tagen bei RT resp. 2-5 Tagen bei 5°C | nicht bestimmt |
| Benzyl Alkohol 0,9% | deutliche Partikelbildung (Wirbel aus dem Vialboden) nach 1-2 Tagen bei RT resp. 2-5 Tagen bei 5°C | nicht bestimmt |
| Solvens enthaltend Natriumglycocholat und Lezithin [1] | klare Lösung. Keine Partikelbildung bis mindestens 1 Monat nach Herstellung bei RT und 5°C | 106,1 % (1 Mo./RT) resp. 95,2 % (1 Mo./5°C) |

[1] Das verwendete Solvens setzt sich wie folgt zusammen:

| | |
|---|---|
| Glycocholsäure | 88,5 mg |
| NaOH 40 % | 19,0 ml |
| Lezithin | 169,0 mg |
| Benzyl Alkohol | 9,0 mg |
| NaOH 1 N ad pH 6,0 | q.s. |
| Wasser zu Injektionszwecken q..s. ad | 1,0 ml |

Es kann folgendermassen hergestellt werden.

8,85 g Glycocholsäure werden in 50 ml $N_2$-begastem Wasser zu Injektionszwecken suspendiert und mit Hilfe von 1,9 ml NaOH 40% gelöst. 16,9 g fein zerschnittenes Lezithin werden hinzugefügt und durch Rühren gelöst. 0,9 g Benzylalkohol werden zugegeben und die erhaltene Lösung nach Einstellung des pH-Wertes mit NaOH 1 N auf 6,0 mit $N_2$-begastem Wasser zu Injektionszwecken auf 100 ml ergänzt. Diese Lösung wird über ein Membranfilter (0,45 $\mu$m) filtriert, unter aseptischen Bedingungen in Ampullen abgefüllt und schliesslich im Autoklaven sterilisiert.

Beispiel 3

Wegen einer deutlichen Adsorption von rIFN-α an der Wand von Lyophilisationsfläschchen aus Glas führt die Rekonstitution von Humanserumalbumin-freien Lyophilisaten mit 1 Mio. I.E. r-IFN-α mit dem üblicherweise verwendeten Wasser zu Injektionszwecken zu Lösungen, die einen deutlich niedrigeren Gehalt an rIFN-α zeigen. Wie aus Tabelle 4 ersichtlich ist, bewirkt ein Solvens mit Natriumglycocholat und Lezithin die gewünschte Desorption von rIFN-α. Die Verabreichung genauer Dosen von rIFN-α wird somit ermöglicht, ohne auf die Anwendung des problematischen Humanserumalbumins zurückgreifen zu müssen.

Wenn gewünscht, können Natriumglycocholat und Lezithin der zu lyoptulisierenden Lösung von rIFN-α oder auch einer gebrauchsfertigen Ampullenlösung von rIFN-α zugesetzt werden, um auf diese Weise der Adsorption des Proteins an der Glaswand der Vials vorzubeugen.

Tabelle IV

| Antivirale Aktivität von rIFN-α-Lösungen aus Humanserumalbumin-freien Lyophilisaten mit 1 Mio I.E. rIFN-α und 1 ml Solvens. | |
| --- | --- |
| **Solvens** | **Antivirale Aktivität** |
| Wasser zu Injektionszwecken | 0,57 Mio I.E. |
| Solvens Nr. 1 [1] | 0,91 Mio I.E. |
| Solvens Nr. 2 [1] | 0,96 Mio I.E. |

1) Die 2 Solventien wurden aus dem unter Tabelle III beschriebenen Solvens durch Verdünnung mit steriler Glucose 5% 1 + 19 (Solvens Nr. 1) resp. 1 + 3 (Solvens Nr. 2) hergestellt. Sie enthalten pro ml 4,4 mg resp. 22,1 mg Natriumglycocholat und 8,45 mg resp. 42,25 mg Lezithin.

**Patentansprüche**

1. Verwendung eines Gallensäuresalzes und eines Lipids zur Adsorptionsverhinderung, Desorption, Agglomerationsverhinderung und Desagglomerierung von immunmodulatorisch wirksamen Proteinen in wässrigen Lösungen.

2. Verwendung nach Anspruch 1, worin das immunmodulatorisch wirksame Protein ein Wachstums- oder Differenzierungsfaktor ist.

3. Verwendung nach Anspruch 2, worin der Wachstums- oder Differenzierungsfaktor ein Colony Stimulating Factor ist.

4. Verwendung nach Anspruch 1, worin das immunmodulatorisch wirksame Protein ein Protein zur Herstellung von Vakzinen ist.

5. Verwendung nach Anspruch 1, worin das immunmodulatorisch wirksame Protein ein Cytokin ist.

6. Verwendung nach Anspruch 5, worin das Cytokin ein Interferon oder Interleukin ist.

7. Verwendung nach Anspruch 5, worin das Cytokin rIFH-α, rIFN-β oder IL-2 ist.

8. Verwendung nach den Ansprüchen 1-7 zur parenteralen und enteralen Verabreichung.

**Claims**

1. Use of a cholanic acid salt and a lipid for adsorption prevention, desorption, agglomeration prevention and deagglomeration of proteins having immunomodulatory activity in aqueous solutions.

2. Use according to claim 1, wherein the protein having immunomodulatory activity is a growth or differentiation factor.

3. Use according to claim 2, wherein the growth or differentiation factor is a colony stimulating factor.

4. Use according to claim 1, wherein the protein having immunomodulatory activity is a protein for the production of vaccines.

5. Use according to claim 1, wherein the protein having immunomodulatory activity is a cytokine.

6. Use according to claim 5, wherein the cytokine is an interferon or interleukin.

7. Use according to claim 5, wherein the cytokine is rIFH-$\alpha$, rIFN-$\beta$ or IL-2.

8. Use according to claims 1-7 for parenteral and enteral administration.

**Revendications**

1. Utilisation d'un acide biliaire et d'un lipide pour la prévention de l'adsorption, la désorption, la prévention de l'agglomération et la désagglomération de protéines actives immunomodulatrices dans des solutions aqueuses.

2. Utilisation selon la revendication 1, dans laquelle la protéine efficace immunomodulatrice est un facteur de croissance ou de différenciation.

3. Utilisation selon la revendication 2, dans laquelle le facteur de croissance ou de différenciation est un facteur stimulant de colonies.

4. Utilisation selon la revendication 1, dans laquelle la protéine active immunomodulatrice est une protéine pour la fabrication de vaccins.

5. Utilisation selon la revendication 1, dans laquelle la protéine active immunomodulatrice est une cytokine.

6. Utilisation selon la revendication 5, dans laquelle la cytokine est un interféron ou une interleukine.

7. Utilisation selon la revendication 5, dans laquelle la cytokine est rIFN-$\alpha$, rIFN-$\beta$ ou $IL-2$.

8. Utilisation selon les revendications 1 à 7 pour l'administration parentérale et entérale.